Europäisches Patentamt

European Patent Office

Office européen des brevets

(11) Publication number: **0 397 103**
**A1**

## (12) EUROPEAN PATENT APPLICATION

(21) Application number: **90108642.1**

(22) Date of filing: **08.05.90**

(51) Int. Cl.⁵: **A61M 5/50, A61M 5/32**

(30) Priority: **11.05.89 KR 896355**

(43) Date of publication of application:
**14.11.90 Bulletin 90/46**

(84) Designated Contracting States:
**DE FR GB**

(71) Applicant: **Kim, Kyu-Hwan**
**Boram APT 205-309, 639, Sangkye-dong**
**Nowon-ku, Seoul(KR)**

(72) Inventor: **Kim, Kyu-Hwan**
**Boram APT 205-309, 639, Sangkye-dong**
**Nowon-ku, Seoul(KR)**

(74) Representative: **Patentanwälte Grünecker,**
**Kinkeldey, Stockmair & Partner**
**Maximilianstrasse 58**
**D-8000 München 22(DE)**

(54) **Disposable injection syringe.**

(57) A disposable injection syringe includes a gasket (7') having a passage (8) for slidably receiving an arrow pin (13), and a plunger (17) having a plunger head (16) for operatively locking the arrow pin (13) and a plurality of apertures (19). An alternative embodiment further includes a needle hub having a conical groove and a syringe tip having a conical projecting member for tightly engaging with the conical groove, whereby neither the injection syringe nor its needle alone can be reused after a first used.

**FIG.11**

**FIG.15**

EP 0 397 103 A1

# DISPOSABLE INJECTION SYRINGE

## BACKGROUND OF THE INVENTION

### 1. Field of the Invention

The present invention relates to a disposable injection syringe which prevents reuse thereof.

### 2. Description of the Prior Art

Various types of disposable injection syringes are well known in the art. However, such disposable syringes have number of problems such as, for example, (1) it is possible to intentionally use the injection syringe repeatedly by injecting a plurality of small quantities of the syringe contents into several different persons; (2) it is difficult to determine if the disposable injection syringe has already been used or not. Therefore, if the used injection syringe is reused, it may be dangerously contaminated; and (3) requirements for managing and supervising the safekeeping and control of the disposal of used injection syringes result in extra expenses.

## SUMMARY OF THE INVENTION

Accordingly, it is an object of the present invention to provide an improved disposable injection syringe having a structure with a syringe tip which prevents the needle from being reused.

Another object of the present invention is to provide a disposable injection syringe which is easily examined with the naked eye to determine whether it has been previously used.

A further object of the present invention is to provide a disposable injection syringe which does not require extra expenses for the disposal thereof.

Other objects and further scope of applicability of the present invention will become apparent from the detailed description given hereinafter. It should be understood, however, that the detailed description and specific examples, while indicating preferred embodiments of the invention, are given by way of illustration only, since various changes and modifications within the spirit and scope of the invention will become apparent to those skilled in the art from this detailed description.

Briefly described, the present invention relates to a disposable injection syringe which includes a gasket having a passage for slidably receiving an arrow pin, and a plunger having a plunger head for operatively locking the arrow pin and a plurality of apertures, as well as to a syringe having a needle hub having a conical groove and a syringe tip having a conical projecting member for tightly engaging with the conical groove, whereby the injection syringe can only be used once and cannot be reused.

## BRIEF DESCRIPTION OF THE DRAWINGS

The present invention will become more fully understood from the detailed description given hereinbelow and the accompanying drawings which are given by way of illustration only, and thus are not limitative of the present invention, and wherein:

Fig. 1 is a sectional view of a needle and a needle hub of a conventional disposable injection syringe;

Fig. 2 is a sectional view of a needle and a needle hub of the disposable injection syringe according to the present invention;

Fig. 3 is a sectional view showing a syringe tip of the conventional disposable injection syringe;

Fig. 4(A) is a sectional view of the syringe tip according to the present invention;

Figs. 4(B) and 4(C) are perspective and top plan views of the syringe tip of the present invention;

Fig. 5(A) is a sectional view of a plunger gasket of the conventional disposable injection syringe;

Fig. 5(B) is a perspective view of Fig. 5(A);

Fig. 6(A) is a sectional view of a plunger gasket of the present invention;

Fig. 6(B) is a perspective view of Fig. 6(A); ·

Figs. 7(A) and 7(B) are plan, and perspective views of an arrow pin which is inserted into a tunnel of the present invention;

Fig. 8 is a top plan view of a plunger head and a head of plunger body of the conventional disposable injection syringe;

Fig. 9 is a sectional view of the plunger head and the plunger body of the present invention;

Fig. 10 is a perspective view of the plunger head of the present invention;

Fig. 11 through Fig. 17 are sectional views illustrating the operational state of the gasket and the plunger body in sequence according to the present invention;

Figs. 18(A) and 18(B) are sectional views of another embodiment of the plunger gasket of the present invention;

Fig. 19 is a sectional view of another embodiment of the plunger body of the second embodiment;

Figs. 20 (A), 20 (B), and 20(C) are views of Fig. 19 , taken along line I-I, J-J, and K-K, respectively; and

Figs. 21 (A), 21 (B), 21(C), and 21 (D) are sectional views illustrating the operational state of the gasket and the plunger body in sequence according to another embodiment of the present invention.

## DESCRIPTION OF THE PREFERRED EMBODIMENTS

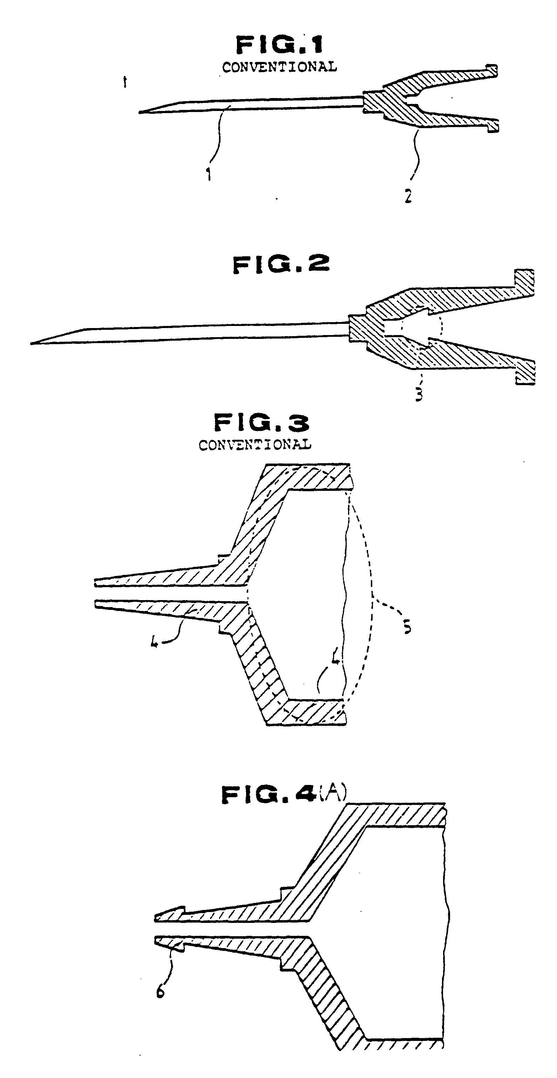

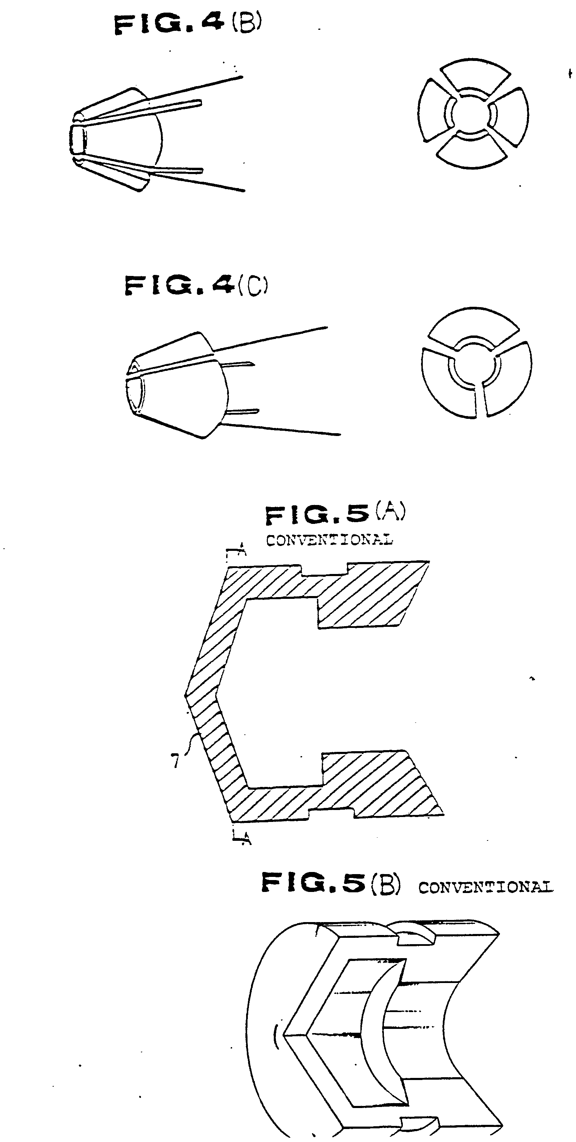

Referring now in detail to the drawings for the purpose of illustrating preferred embodiments of the present invention, the disposable injection syringe as shown in Figs. 2, 4(A), 4(B), and 4(C) comprises a needle 1, a needle hub 2, and the syringe tip 4. On the other hand, Figs. 1 and 3 show a conventional injection syringe wherein the syringe tip 4 is inserted into an engaging groove of the needle hub 2 such that the needle 1 is available for reuse. The syringe tip 4 has a conical projecting member 6 disposed at the front end thereof. Also, the needle hub 2 has a conical engaging groove 3 for tightly engaging with the conical projecting member 6 so as to prevent the syringe from separating from the needle hub 2 and thereby prevent the needle 1 from being reused.

As shown in Figs. 4(B) and 4(C), the conical projecting member 6 according to the present invention is formed like a collet chuck. The conical projecting member 6 has a plurality of slits, such as three or four slits, disposed along the surface thereof. This allows it to be easily inserted and makes it almost impossible to be separated after being inserted.

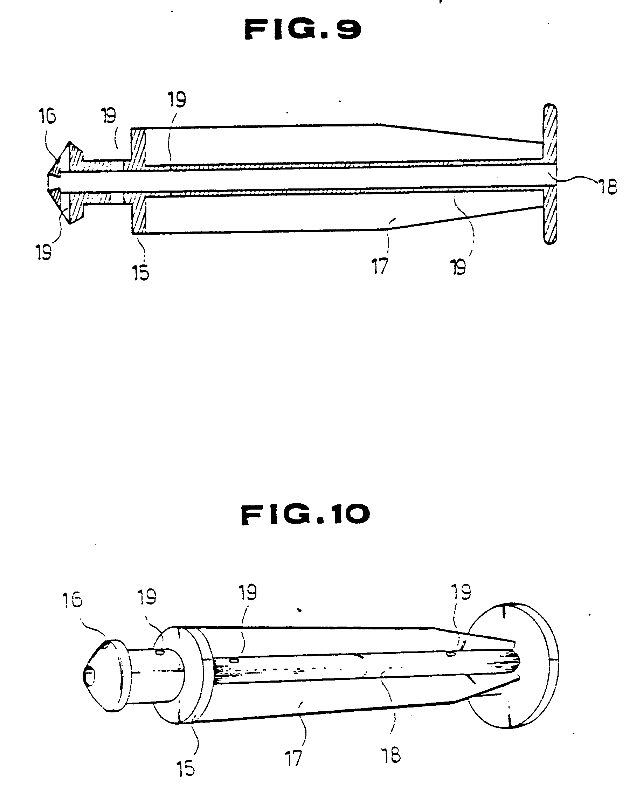

Figs. 5(A) is a sectional views of a gasket 7 of a conventional syringe which is used to seal the syringe when the plunger body reciprocates along the side of the syringe body 4' as the plunger head 14 (note fig. 8) is inserted into the side of the gasket.

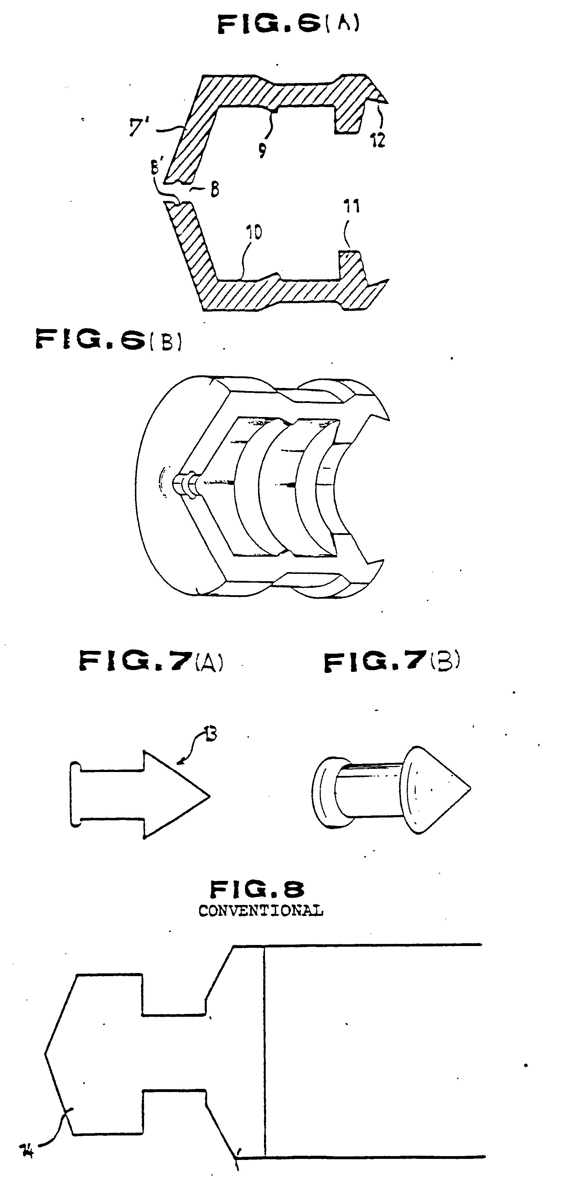

Figs. 6 (A) 6(B) show the gasket 7' of the present invention which includes a passage 8 disposed at the front portion of a gasket head for passing air or liquid therethrough and a stopper 9 for discharging trapped air together with the injection substance from the syringe. An arrow pin 13 as shown in Fig. 7(A) is inserted into the passage 8. At that time, an annular raised portion of the arrow pin 13 is inserted into a circular groove 8' disposed at the gasket, in the vicinity of the passage 8. The gasket 7 further includes a sliding area 10 for the plunger head 16 to move forvard or backward therealong in the inside of the gasket 7', a back stopper 11 for stopping the back portion of a plunger head 16, and restitution member 12 for acting as a spring.

When a head of the plunger body 17 as shown in Fig. 9 is forced forward, the restitution member 12 is compressed and again restores to its former state after the force is removed.

As shown in Figs. 9, the plunger body 17 includes a main channel 18 for freely passing in liquid and an auxiliary channel 19 roundly connected to the main channel 18. The plunger head 16 contains a locking member 16' for falling off the arrow pin 13 inserted into the front portion of the gasket head 7' when the plunger is pulled from the gasket.

The disposable injection syringe according to the present invention operates as follows:

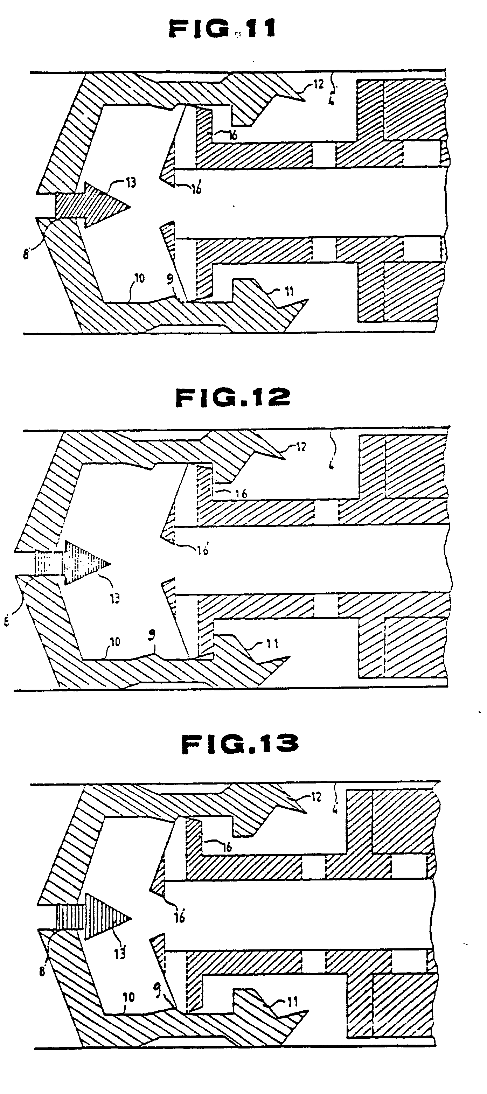

Fig. 11 shows the arrow pin 13 disposed in the gasket head 7' and the plunger head 16 disposed between the stopper 9 and the back stop pin 11.

In this state, when the plunger is pulled to draw a substance into the syringe body 4' as shown in Fig. 12, the plunger head 16 is stopped by the back stopper 11. Thus, when the gasket is to be pushed out, air pressure inside the syringe body 4' falls rapidly and the substance to be contained in the syringe is drawn into the syringe.

At this time, the air pressure inside of the gasket adjusts to atmospheric pressure since the channel 18 and the auxiliary channel 19 (Fig. 9 ) are cylindrically connected to the inside of the syringe body 4'.

In order to discharge the air which is also drawn into the syringe with the substance contained in the syringe, when the plunger is steadily pushed into the gasket as shown in Fig. 13, the plunger head 16 slips up from the back stopper 11 and force is added to the stopper 9, and thus the gasket moves forward. At this time, air drawn in with the syringe contents is discharged to the outside through the needle 1. The forces given to the plunger can be divided into a force ($\alpha$) and a force ($\beta$), wherein $\alpha > \beta$, and the force ($\alpha$) is the force required for the plunger to be pushed into the front of the syringe in order to discharge air drawn in with the syringe contents, and the force ($\beta$) is the force required for the plunger to be pushed to the front of the syringe to inject liquid into muscles or veins. The force which is needed to inject into muscles and veins is greater than the force required for the plunger to discharge air drawn into the syringe from the atmosphere.

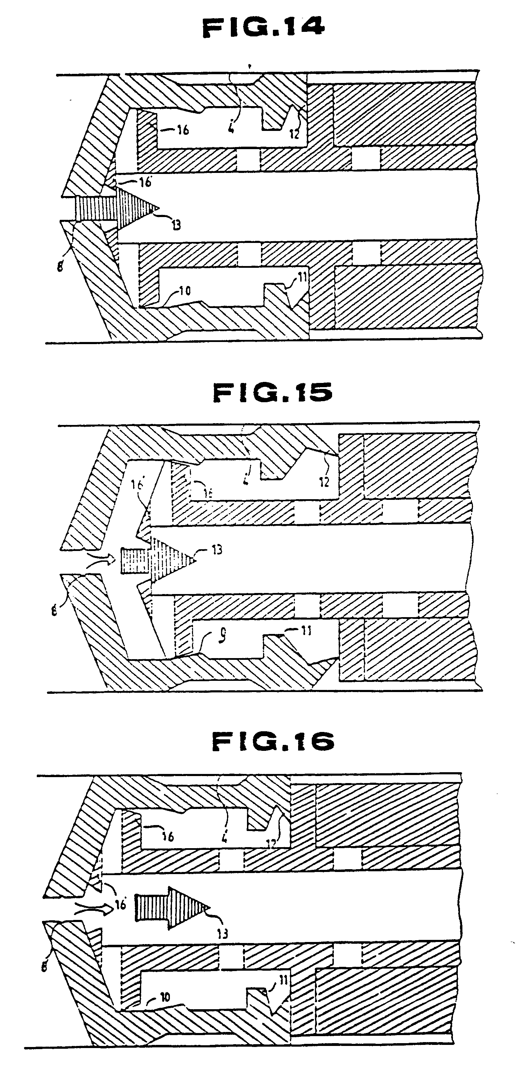

After discharging entirely the air drawn into the syringe toward the atmosphere so as to be able to inject liquid into the human body, the plunger is pushed up with greater force than that required for discharging air which moves the plunger to a state as shown in Fig. 14 wherein the plunger head 16 passes over the back stopper 11 disposed in the center of the inside of the gasket and slides over a sliding area 10. At the same time, the plunger head 16 comes to be positioned closely to the front portion of the inside of the gasket and the arrow pin 13 inserted into the gasket is easily inserted into the locking member 16' disposed in the front portion of the plunger head for compressing restitution member 12 by the head 15 of the plunger body 17.

At this moment, when the passage 8 of the gasket head 7' is closed by the arrow pin 13, the contents in the syringe are injected into the human body through the needle 1.

Fig. 15 shows the state of the syringe after the injection is finished or stopped part way. When the plunger is continuously pushed up and is stopped, at the very moment, the restitution member 12 is pushed the head 15 of the plunger body 17. In order to be restored to the original state, the plunger is pushed backward and simultaneously the gasket is pulled backward so that the arrow pin 13 inserted into the passage 8 of the gasket head 7' through locking member 16' formed in the plunger head 16 is released therefrom.

When the arrow pin 13 is moved out, since the inside of the gasket and the syringe body 4' are connected through the tunnel 8, the air pressure inside the syringe adjusts to that of the atmosphere.

When the plunger is pushed up to inject liquid into the human body again, high pressure is added to the injection so that the injection is pushed into the inside of human body through the needle 1 as shown in Fig. 16. The arrow pin 13 is pushed into the passage 8 of the inside of the plunger so that the injection spouts from the passage 8 of the gasket head 18. Therefore, it is impossible to inject a small quantity into several different persons with the syringe.

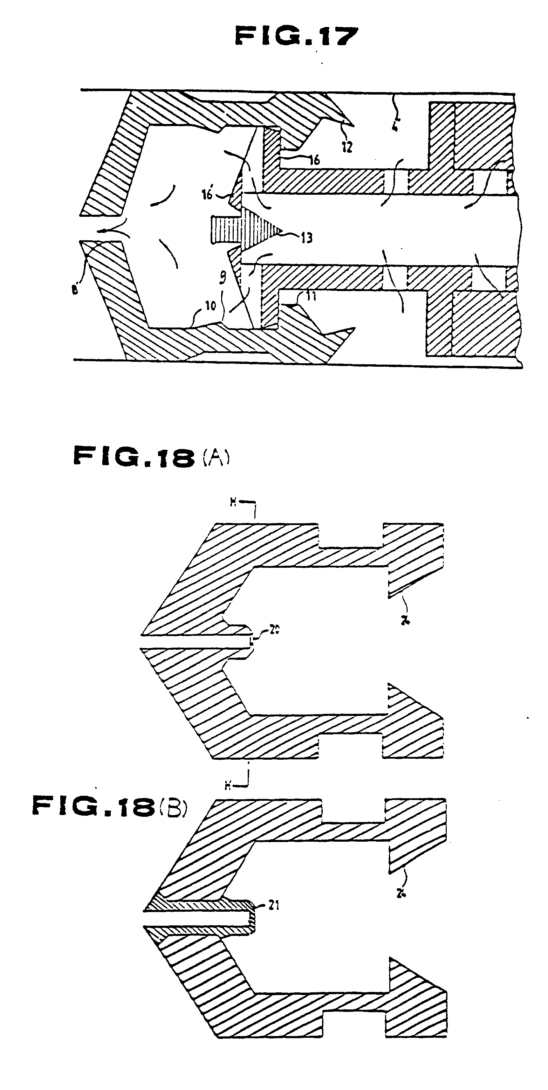

After the injection is wholly exhausted, and the plunger is pulled up in an attempt to draw a substance into the syringe as shown in Fig. 17, air and liquid in the plunger body 17 and the plunger head 16 passes freely through the channel 18 an auxiliary channel 19. At that time, air in the front of the inside of the gasket is drawn into the syringe through the passage 8. Therefore, the pressure of the inner syringe is adjusted to that of the atmosphere and so that injecting is inhaled again. That is, it is made impossible for a substance to be drawn in again into the syringe after it has been used once already.

Fig. 18(A) through Fig. 21(D) show another embodiment of the present invention.

Fig. 18(A) shows the gasket when a substance is drawn into the syringe. A thin part 20 which is endurable to inhalant power is positioned in the front part of the gasket and a back stopper 24 is located in the rear. Fig. 18(B) shows the gasket including a separate element 21 which serves as the thin part.

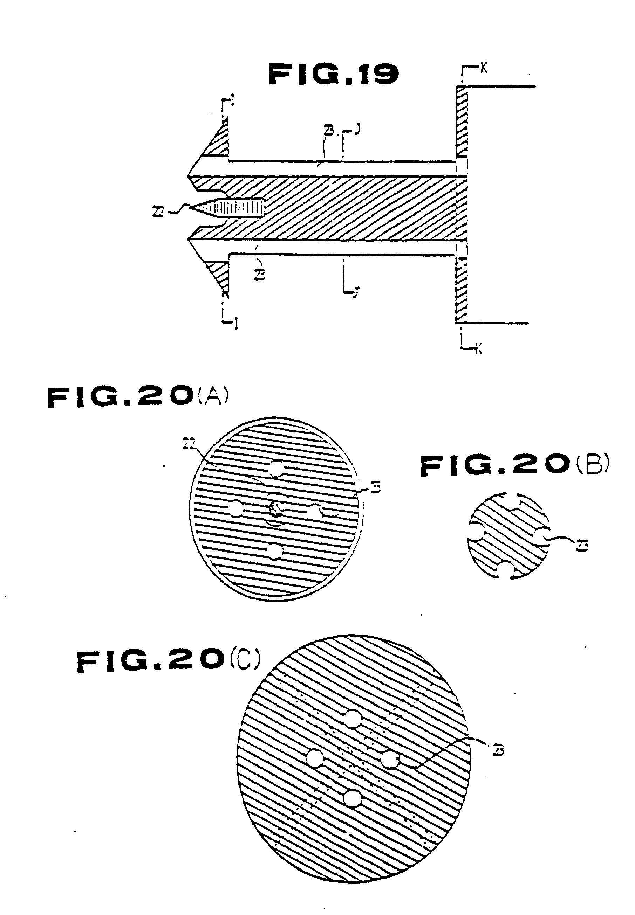

Fig. 19 shows the plunger which has a tunnel 23, and a pin 22 for penetrating the thin part of the gasket shown in Figs. 18(A) and 18(B).

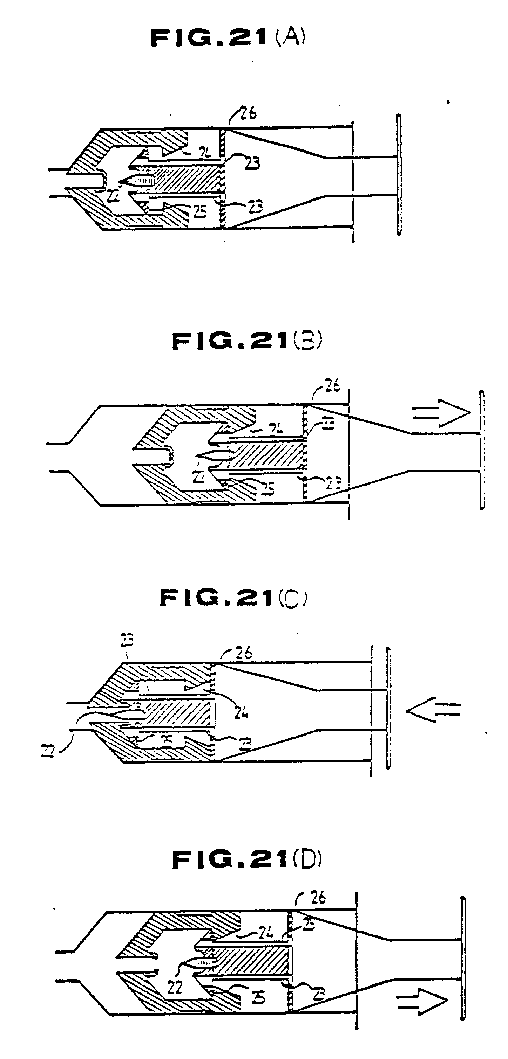

In operation, Fig. 21(A) shows a state in which part of the inside of the syringe body is assembled and the plunger head is located inside the gasket. When the plunger is pulled up to draw a substance into the syringe as shown in Fig. 21(B), the back part of the plunger head 25 is positioned closely to a back stopper 24, the gasket and the plunger move along together backward, and the liquid to be injected is drawn into the syringe.

In this state, when the plunger moves forward to inject liquid into the human body as shown in Fig. 21(C), the pin 22 penetrates the thin part 20 of the gasket. When the plunger is constantly pushed up, the injection is accomplished and the liquid is injected into the human body.

As shown in Fig. 21C), after the liquid is entirely exhausted, when the plunger is pulled up as shown in Fig. 21(D), more liquid is again attempted to be drawn into the syringe, the front inside of gasket is full of air drawn into the syringe through the tunnel 23 which is provided in the plunger body and the plunger head so that the air pressure inside the syringe becomes that of the atmosphere. Therefore, it is impossible for liquid to be drawn into the syringe again. That is, it is impossible for liquid to be drawn into the syringe after it has already been used once.

Accordingly, the disposable injection syringe of the present invention can only be used once and it is impossible to reuse it again. The substance to be injected is drawn into the syringe and injected even if syringe which is extruded unfairly after using is paved and settled not to discriminate, by appearance, whether it is used or not, so it is effective that the well-intended user can confirm, easily whether it is used or not by the naked eye and other simple method.

Also, in the case wherein medical staff uses the syringe of the present invention, the patient can easily confirm with the naked eye that the syringe has been used, so it makes it possible, for confidence to grow between medical staff and patients since the risk of spreading infection from a used syringe is greatly reduced. Public confidence in the administration of injection is also improved since the syringe can only be used once.

The invention being thus described, it will be obvious that the same may be varied in many ways. Such variations are not to be regarded as a departure from the spirit and scope of the invention, and all such modifications as would be obvious to one skilled in the art are intended to be included in the scope of the following claims.

## Claims

1. A disposable injection syringe comprising:
a gasket including a passage disposed at the front portion thereof, a first stopper and a second stopper disposed in the inside thereof and a restitution member disposed at the rear portion thereof,
an arrow pin slidably disposed in said passage,
a plunger including a plunger head slidably disposed between said first stopper and said second stopper, a head edge extending from said head for operatively locking with said arrow pin, and
a plurality of vertical apertures for communicating with the inside of the injection syringe whereby the disposable injection syringe can only be used once and cannot be reused.

2. The disposable injection syringe of claim 1, wherein the passage is provided with a circular groove disposed at the head of the gasket for slidably receiving an annular raised portion of a body of the arrow pin.

3. The disposable injection syringe of claim 1, wherein the plurality of vertical apertures comprise a main aperture disposed in a plunger body and an auxiliary aperture disposed in the plunger head, respectively.

4. A disposable injection syringe comprising:
a gasket including a passage disposed at the front portion thereof, and a thin cover extending from said gasket for covering end of said passage, and
a plunger including a pin disposed at the front center portion thereof for tearing said thin cover and a plurality of latitudinal apertures for communicating with the inside of the injection syringe, whereby the disposable injection syringe cannot be reused after it is used once.

5. The disposable injection syringe of claim 4, wherein the thin cover is separate member from the gasket.

6. A disposable injecting syringe comprising:
a needle,
a needle hub supporting to said needle, said needle hub including a conical groove disposed therein, and
a syringe tip extending from the injection syringe, said syringe tip including a conical projecting member for tightly engaging with said conical groove, whereby the needle of the disposable injection syringe cannot be reused after it is used once.

7. The disposable injection syringe of claim 6, wherein the conical projecting member is composed of a plurality of slits.

8. The disposable injection syringe of claim 7, wherein the plurality of slits are three.

9. The disposable injection syringe of claim 7, wherein the plurality of slits are four.

# FIG.1
CONVENTIONAL

# FIG.2

# FIG.3
CONVENTIONAL

# FIG.4(A)

# FIG.4 (B)

# FIG.4 (C)

# FIG.5 (A)
CONVENTIONAL

7

# FIG.5 (B) CONVENTIONAL

## FIG.6(A)

## FIG.6(B)

## FIG.7(A)          FIG.7(B)

## FIG.8
CONVENTIONAL

# FIG.9

# FIG.10

# FIG.11

# FIG.12

# FIG.13

# FIG.14

# FIG.15

# FIG.16

# FIG.17

# FIG.18 (A)

# FIG.18 (B)

# FIG.19

# FIG.20(A)

# FIG.20(B)

# FIG.20(C)

## FIG.21(A)

## FIG.21(B)

## FIG.21(C)

## FIG.21(D)

## DOCUMENTS CONSIDERED TO BE RELEVANT

EP 90108642.1

| Category | Citation of document with indication, where appropriate, of relevant passages | Relevant to claim | CLASSIFICATION OF THE APPLICATION (Int Cl⁴) |
|---|---|---|---|
| A | <u>EP - A1 - 0 300 694</u><br>(DOWTY SEALS LTD.)<br>  * Fig. 1-4,12-14; column 6,<br>    line 18 - column 7, line 6;<br>    column 7, line 20 - column 8,<br>    line 10; column 10, line 47<br>    - column 11, line 55 *<br>    -- | 1,4 | A 61 M 5/50<br>A 61 M 5/32 |
| A | <u>EP - A1 - 0 291 109</u><br>(NV MEDICOPHARMA)<br>  * Fig. 1A-1C; column 4,<br>    line 33 - column 5, line 9;<br>    column 5, line 32 - column 6,<br>    line 23 *<br>    -- | 4 | |
| A | <u>US - A - 4 687 467</u><br>(B. CYGIELSKI)<br>  * Fig. 1,3,4; abstract;<br>    column 2, lines 32-46;<br>    column 2, line 64 - column 3,<br>    line 2 *<br>    -- | 4 | |
| X | <u>CH - A5 - 585 560</u><br>(ASICAN A/S)<br>  * Fig. 5; column 3, lines<br>    17-25 *<br>    -- | 6 | TECHNICAL FIELDS SEARCHED (Int Cl⁴)<br><br>A 61 M |
| A | <u>US - A - 4 281 653</u><br>(H. BARTA et al.)<br>  * Fig. 1-3; column 2, line<br>    60 - column 3, line 22 *<br>    ---- | 6 | |

The present search report has been drawn up for all claims

| Place of search | Date of completion of the search | Examiner |
|---|---|---|
| VIENNA | 14-08-1990 | LUDWIG |